# EUROPEAN PATENT SPECIFICATION

(11) **EP 3 072 529 B1**
(45) Date of publication and mention of the grant of the patent: **05.07.2017**
(21) Application number: 15160958.3
(22) Date of filing: 26.03.2015
(51) Int. Cl.: A61K 47/38, A61K 9/16, A61K 47/00, A61K 9/00, A61K 31/437

(54) **COMPOSITION COMPRISING VEMURAFENIB AND HPMC-AS**
ZUSAMMENSETZUNG MIT VEMURAFENIB UND HPMC-AS
COMPOSITION COMPRENANT DU VEMURAFENIB ET DU HPMC-AS

(43) Date of publication of application: 28.09.2016
(73) Proprietor: ratiopharm GmbH, 89079 Ulm (DE)
(72) Inventor: Lehmann, Frank, 90233 Neu-Ulm (DE); Guserle, Richard, 89359 Kötz (DE); Albrecht, Wolfgang, 89075 Ulm (DE)
(74) Representative: Ter Meer Steinmeister & Partner

(56) References cited:
- WO-A2-2010/114928
- US-A1- 2012 148 533
- "Zelboraf, Vemurafenib assessment report", , 15 December 2011 (2011-12-15), XP055202871, Retrieved from the Internet: URL:http://www.ema.europa.eu/docs/en_GB/do cument_library/EPAR_-_Public_assessment_re port/human/002409/WC500124400.pdf [retrieved on 2015-07-16]
- RICHARD J BASTIN ET AL: "Salt Selection and Optimisation Procedures for Pharmaceutical New Chemical Entities", ORGANIC PROCESS RESEARCH AND DEVELOPMENT, AMERICAN CHEMICAL SOCIETY, US, vol. 4, no. 5, 19 July 2000 (2000-07-19), pages 427-435, XP008154792, ISSN: 1083-6160, DOI: 10.1021/OP000018U [retrieved on 2000-07-19]
- NAVNIT SHAH ET AL: "Improved human bioavailability of vemurafenib, a practically insoluble drug, using an amorphous polymer-stabilized solid dispersion prepared by a solvent-controlled coprecipitation process", JOURNAL OF PHARMACEUTICAL SCIENCES, vol. 102, no. 3, 29 March 2013 (2013-03-29), pages 967-981, XP055112594, ISSN: 0022-3549, DOI: 10.1002/jps.23425

## Description

### Background of the invention

The present invention relates to a premix comprising vemurafenib, in particular vemurafenib hydrochloride, and hydroxypropyl methyl cellulose acetate succinate in a specific weight ratio and to oral dosage forms comprising said premix. Further, the invention relates to a process for producing said premix comprising vemurafenib hydrochloride and HPMC-AS and to the corresponding process of producing an oral dosage form containing the premix of the invention. Further, the invention relates to a method for treating different types of cancer, such as colorectal cancer, ovarian carcinoma, thyroid cancer and malignant melanoma, in particular malignant melanoma, comprising administering the dosage form comprising the premix of the invention.

### Technical Background

Vemurafenib is an active pharmaceutical ingredient which is reported to be a potent and selective B-Raf kinase inhibitor. In particular, vemurafenib specifically inhibits the V600E mutation of B-Raf, which has been implicated in various tumours, for example colorectal cancer, ovarian carcinoma, thyroid cancer and malignant melanoma. It is reported that in xenoengrafted malignant melanomas B-Raf could be deactivated by vemurafenib and a regression of the tumor(s) could be achieved. Said mentioned V600E mutation in B-Raf is reported to be present in about 50% of the human malignant melanoma such that vemurafenib is effective to the genotype.

In the context of this invention, the term "vemurafenib" refers to propane-1-sulfonic acid {3-[5-(4-chlorophenyl)-1H-pyrrolo[2,3-b]pyridine-3-carbonyl]-2,4-difluorophenyl}-amide according to Formula (1).

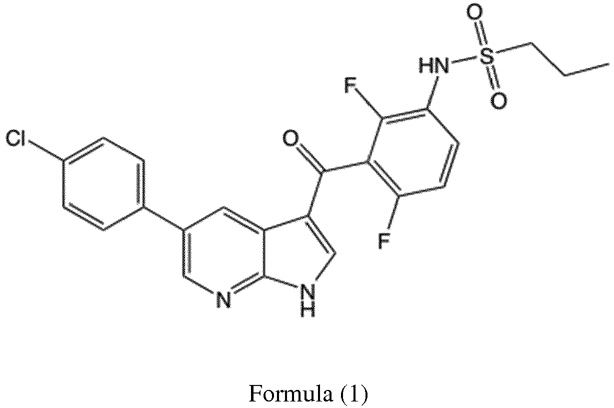

Vemurafenib base is reported to be a BCS IV compound and practically insoluble in aqueous and physiological solvents. Further, the HCl salt is reported to be better soluble.

US 2012/148533 A1 relates to a combination therapy of propane-1-sulfonic acid{3-[5-(4-chloro-phenyl)-1H-pyrrolo[2,3-b]pyridine-3-carbonyl-2,4-difluoro-phenyl]-amide}, or a pharmaceutically acceptable salt thereof, and an interferon for treating a patient suffering from a proliferative disorder, in particular a solid tumor, for example, colorectal cancer, melanoma, and thyroid cancer. In particular, the application relates to such a therapy wherein the interferon is peginterferon alfa-2a and the disorder is melanoma containing the V600E b-Raf mutation.

WO 2010/114928 A2 relates to solid dispersions, solid molecular complexes, salts and crystalline polymorphs of vemurafenib. In particular WO 2010/114928 A2 relates to solid dispersions comprising vemurafenib molecularly dispersed within a polymer matrix of an ionic polymer. In this solid dispersion the active agent is considered to be primarily in amorphous form.

The teaching of WO 2010/114928 was further developed in WO 2011/057974 A1. Said application discloses a method for manufacturing a solid dispersion containing amorphous vemurafenib and hydroxypropyl methyl cellulose acetate succinate, hereinafter referred to as HPMC-AS. Said method results in a solid dispersion wherein the solid dispersion subsequently can be further used to prepare an oral dosage form. This oral dosage form is supposed to be the market product Zelboraf^{®} (see "Zelboraf, Vemurafenib assessment report", 15 December 2011, retrieved form the internet: ema.europa.eu/docs/en_GB/document_library/EPAR_-_Public_assessment_report/human/002409/WC500124400.pdf). Zelboraf^{®} is a tablet inter alia containing 240 mg vemurafenib and 560 mg HPMC-AS and is usually administered to a patient in a regime of 960 mg active agent (four tablets) twice a day.

However, a tablet having a weight of about 870 mg would be undesirably large in size and therefore would be difficult to swallow. In particular older people might show a poor patient compliance due to the size of the tablet.

In addition, the patient compliance might be negatively effected by the high number of tablets to be taken each day.

The article by Richard J. Bastin et al., "Salt Selection and Optimisation Procedures for Pharmaceutical New Chemical Entities", ORGANIC PROCESS RESEARCH AND DEVELOPMENT, AMERICAN CHEMICAL SOCIETY, US, vol. 4, no. 5, 19 July 2000, pages 427-435, refers to the selection of an appropriate salt form for a new chemical entity.

The article by Navnit Shah et al., "Improved human bioavailability of vemurafenib, a practically insoluble drug, using an amorphous polymer-stabilized solid dispersion prepared by a solvent-controlled coprecipitation process", JOURNAL OF PHARMACEUTICAL SCIENCES, vol. 102, no. 3, 29 March 2013, pages 967-981, deals with the improvement of the solubility of the practically insoluble drug vemurafenib by converting it into an amorphous-solid dispersion using a solvent-controlled precipitation process.

Hence, it was an object of the present invention to overcome the above problems.

An object of the present invention is to provide a dosage form, preferably a tablet, containing vemurafenib with dissolution properties similar or equal to the ones of Zelboraf^{®}, but having a higher drug-load, such that the dosage form is therefore smaller in size than Zelboraf^{®} potentially leading to a superior patient compliance.

Additionally or alternatively a dosage form containing vemurafenib with dissolution properties similar or equal to the ones of Zelboraf^{®} should be provided which can be administered in a smaller number potentially leading to a better patient compliance as well.

Another object of the invention is the avoidance of organic solvents in the production process. The above-mentioned patent applications, WO 2010/114928 A2 and WO 2011/057974, both use organic solvents in the described formulation processes. However, it has been extensively described that high amounts of organic solvents are harmful to the environment and the employees of the producing companies. Thus, processes using such solvents require higher investments in protective measurements and should therefore be avoided.

### Summary of the Invention

According to the present invention, the above-mentioned technical problems can be solved by a premix comprising vemurafenib hydrochloride and HPMC-AS, wherein vemurafenib and HPMC-AS are present in a specific weight ratio. The premix can advantageously be processed into oral dosage forms of a size or number to be administered more comfortable to the patient.

Thus, the subject of the invention is a premix comprising
a) vemurafenib hydrochloride and
b) HPMC-AS,
wherein the weight ratio of vemurafenib - calculated as free base - to HPMC-AS is from 1:1 to 2:1.

In case the weight of vemurafenib hydrochloride is mentioned in the present invention, this refers to the weight of vemurafenib in form of the free base. Thus, for example, 100 mg of vemurafenib hydrochloride corresponds to 93.1 mg vemurafenib in form of the free base.

A further subject of the present invention is an oral dosage form comprising the premix of the invention and optionally further pharmaceutical excipient(s).

Another subject of the present invention is a method of preparing the premix according to the invention comprising the steps of
(i) providing vemurafenib hydrochloride and HPMC-AS,
(ii) milling the mixture of step (i) and
(iii) optionally sieving the mixture of step (ii).

Further, the subject of the present invention relates to a process for preparing an oral dosage form comprising the steps of
(i) providing vemurafenib hydrochloride and HPMC-AS,
(ii) milling the mixture of step (i),
(iii) optionally sieving the mixture of step (ii),
(iv) optionally adding further excipient(s) to the mixture of step (ii) or step (iii),
(v) optionally granulating the mixture of step (ii), step (iii) or step (iv) and
(vi) processing the mixture of step (ii), step (iii) or step (iv) or the granulates of step (v) into an oral dosage form.

The above-illustrated subjects of the present invention are alternative solutions to the above-outlined problems.

### Detailed Description of the Invention

The present invention concerns a premix comprising vemurafenib hydrochloride and HPMC-AS wherein the components are present in a specific weight ratio.

"Vemurafenib hydrochloride" as used in the present application is represented by the following Formula (1a)

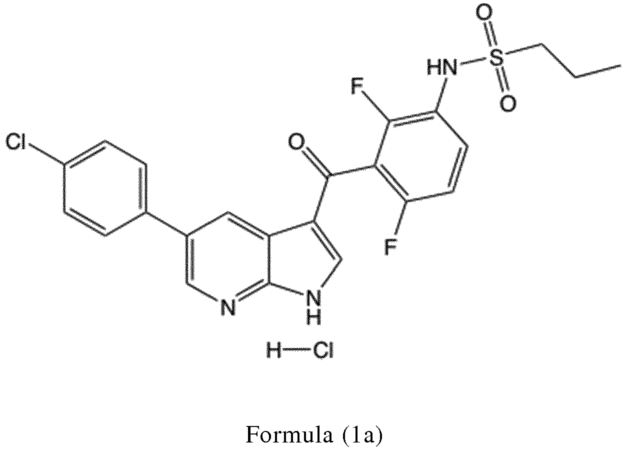

Vemurafenib hydrochloride according to Formula (1a) is particularly preferred. Alternatively, vemurafenib hydrochloride can refer to pharmaceutically acceptable solvates, hydrates and mixtures thereof.

In a particularly preferred embodiment the premix of the present invention as well as the oral dosage form of the present invention comprise vemurafenib hydrochloride as the sole pharmaceutically active agent.

In an alternative embodiment the composition of the present invention as well as the oral dosage form of the present invention can comprise vemurafenib in combination with further pharmaceutically active agent(s).

The premix of the present invention preferably contains vemurafenib hydrochloride in non-crystalline form.

The term "non-crystalline" can be used in the context of this invention to designate a compound in which the components (atoms, ions or molecules) do not exhibit a periodic arrangement over a great range (=long-range order) as usually known from crystalline substances. Consequently, in contrast to crystalline substances, X-ray diffraction performed on non-crystalline substances does not reveal clearly defined interferences for them, but normally only a few diffuse interferences with small diffraction angles.

In a preferred embodiment non-crystalline vemurafenib hydrochloride relates to amorphous vemurafenib hydrochloride. The term "amorphous" is used in the context of this invention to designate the state of solid substances in which the components (atoms, ions or molecules, i.e. in the case of amorphous vemurafenib hydrochloride the corresponding molecules) do not exhibit any periodic arrangement over a great range (=long-range order). In amorphous substances, the components are usually not arranged in a totally disordered fashion and completely randomly, but are rather distributed in such a way that a certain regularity and similarity to the crystalline state can be observed with regard to the distance from and orientation towards their closest neighbours (=short-range order). Amorphous substances consequently preferably possess a short-range order, but no long-range order. In contrast to anisotropic crystals, solid amorphous substances are isotropic. The amorphous substances can be distinguished experimentally from crystalline substances by means of X-ray diffraction, where the amorphous substances do not reveal clearly defined interferences as mentioned above.

In the context of this invention, the expression "amorphous vemurafenib hydrochloride" preferably refers to a substance which consists of amorphous vemurafenib hydrochloride. Alternatively, "vemurafenib hydrochloride" may also contain small amounts of crystalline vemurafenib hydrochloride components, provided that no defined interferences in X-ray diffraction can be detected. A mixture containing 85 to 99.99% by weight amorphous vemurafenib hydrochloride and 0.01 to 15% crystalline vemurafenib hydrochloride is preferred, more preferably 95 to 99.9% by weight amorphous vemurafenib hydrochloride and 0.1 to 5% crystalline vemurafenib hydrochloride. In a particularly preferred embodiment amorphous vemurafenib hydrochloride does not contain crystalline vemurafenib hydrochloride at all. The crystalline proportion is determined by means of quantitative X-ray diffractometry according to the method of Hermans and Weidinger, wherein porcelain powder can be added as reference.

In a preferred embodiment of the invention the premix can preferably contain vemurafenib hydrochloride in an amount from 250 to 550 mg, preferably 280 to 480 mg, more preferably 340 to 420 mg. As indicated above, 100 mg vemurafenib hydrochloride correspond to 93.2 mg vemurafenib as free base such that the above values of the vemurafenib hydrochloride can be transferred into the corresponding values of vemurafenib as free base.

In a preferred embodiment the premix of the invention can have a particle size of 0.1 to 100 µm, preferably 1 to 75 µm, more preferably 2 to 50 µm, especially 5 to 40 µm. The particle size can be determined by means of the microscope "Leica DM2500P", wherein the premix was dispersed in Halocarbon oil 700. When determining the particle size by microscope the "longest dimension" of the particle is measured.

A further component in the premix is hydroxypropyl methylcellulose acetate succinate, which is referred to as HPMC-AS. HPMC-AS is reported to be a partially esterified derivate of HPMC and to have the following chemical structure represented by Formula (II), 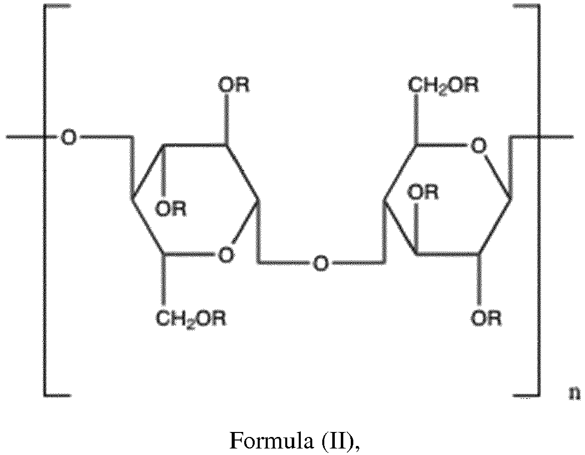 wherein R is selected form H, CH₃, CH₂CH(CH₃)OH, COCH₃, COCH₂CH₂COOH, CH₂CH(CH₃)OCOCH₃ and CH₂CH(CH₃)OCOCH₂CH₂COOH.

In a preferred embodiment HPMC-AS can contain acetyl groups from 2 to 16%, more preferably from 4 to 14%, in particular from 5 to 13%. In a preferred embodiment HPMC-AS can contain succinoyl groups from 4 to 28%, more preferably from 5 to 20%, in particular from 6 to 17 %.

The ratio of acetyl to succinoyl substitution can be from 8:15 to 13:5. It is particularly preferred that the ratio of acetyl to succinoyl substitution can be from 11:7 to 13:5.

In a preferred embodiment, HPMC-AS can have a weight average molecular weight ranging from 5,000 to 300,000 g/mol, preferably from 8,000 to 150,000 g/mol, more preferably from 10,000 to 100,000 g/mol. Furthermore, a 2% w/w solution of HPMC-AS in water at pH 7.0 preferably can have a viscosity of more than 2 mPas, more preferably of more than 5 mPas, particularly more than 8 mPas and up to 850 mPas when measured at 25°C. The viscosity can be determined according to Ph. Eur. 6.0, Chapter 2.2.10. In the above definition the term "solution" may also refer to a partial solution (in case that the polymer does not dissolve completely in the solution). The weight average molecular weight can preferably be determined by gel electrophoresis. In a preferred embodiment the premix of the present invention can preferably comprise 33 to 50 wt%, more preferably 35 to 47 wt% and more preferably 37 to 45 wt% HMPC-AS, based on the total weight of the premix.

In the present invention HMPC-AS can preferably be regarded as a hydrophilic polymer due to its hydrophilic groups, such as hydroxy and carboxy groups. In a preferred embodiment HMPC-AS can enhance the solubility of vemurafenib hydrochloride. Thus, in the present premix HPMS-AS might be regarded as a hydrophilizing agent.

The composition of the present invention can preferably have a weight ratio of vemurafenib as free base to HPMC-AS of 1:1 to 2:1, preferably of 1.01:1 to 1.4:1, more preferably of 1.02:1 to 1.25:1, most preferably of 1.03:1 to 1.15:1.

It turned out that with the above ratio it can be particularly ensured that the complete amount of vemurafenib hydrochloride remains in a non-crystalline state and does reconvert to a crystalline substance.

In a preferred embodiment the premix of the present invention can preferably comprise the following amounts of components:
240 mg to 550 mg vemurafenib hydrochloride, and 110 to 510 mg HPMC-AS, preferably 125 to 445 mg HPMC-AS.

The premix of the present invention can be applied in form of an oral dosage form, in particular in form of a solid oral dosage form. Thus, another object of the present invention is a solid oral dosage form comprising the premix according to the present invention.

In a preferred embodiment the present oral dosage form can preferably comprise further pharmaceutical excipient(s).

The pharmaceutical excipients are excipients with which the person skilled in the art is familiar, such as those which are described in the European Pharmacopoeia (Ph.Eur.) and/or in the US Pharmacopoeia (USP).

In a preferred embodiment of the present invention the oral dosage form can further comprise one or more excipients(s) selected from surfactants (c), fillers (d), binders (e), disintegrants (f), lubricants (g) and glidants (h).

Surfactants (c) can be regarded as substances lowering the interfacial tension between two phases, thus enabling or supporting the formation of dispersions or working as a solubilizer. Common surfactants are alkylsulfates (for example sodium lauryl sulfate), alkyltrimethylammonium salts, alcohol ethoxylates and the like. Surfactants can be used in an amount of 0 to 2 wt%, preferably of 0.1 to 1.5 wt%, based on the total weight of the oral dosage form.

It is particularly preferred that the oral dosage form of the present invention does not contain a surfactant.

Fillers (d) or diluents can be used to increase the bulk volume and weight of a low-dose drug to a limit at which a pharmaceutical dosage form can be formed. Fillers should fulfil several requirements, such as being chemically inert, non-hygroscopic, biocompatible, easily processable and possessing good biopharmaceutical properties. Examples of fillers are lactose, sucrose, glucose, mannitol, calcium carbonate, cellulose and others. Fillers can be used in an amount of 0 to 25 wt%, preferably 1 to 20 wt%, based on the total weight of the dosage form.

It is particularly preferred that the oral dosage form of the present invention does not contain a filler.

Binders (e) may be added to the pharmaceutical formulation in order to ensure that oral dosage forms, preferably tablets, can be formed with the required mechanical strength. The binder can, for example, be starch, polyvinyl pyrrolidone or cellulose derivatives such as hydroxypropyl cellulose. Binders can be present in an amount of 0 to 10 wt%, preferably 0.2 to 8 wt%, more preferably 0.4 to 5 wt%, based on the total weight of the pharmaceutical formulation.

Disintegrants (f) are compounds which enhance the ability of the dosage form, preferably the ability of the tablet to break into smaller fragments when in contact with a liquid, preferably water. Preferred disintegrants are sodium carboxymethyl starch, cross-linked polyvinyl pyrrolidone (crospovidone), sodium carboxymethyl glycolate (for example Explotab^{®}), swelling polysaccharide, for example soy polysaccharide, carrageenan, agar, pectin, starch and derivatives thereof or protein, for example formaldehyde-casein, sodium bicarbonate or mixtures thereof. More preferred are sodium carboxymethyl cellulose and cross-linked polyvinyl pyrrolidone (crospovidone). Disintegrants can be used in an amount of 0 to 15 wt%, preferably of 1 to 12 wt%, more preferably 2.5 to 8 wt%, based on the total weight of the dosage form.

The function of lubricants (g) is reported to ensure that tablet formation and ejection can occur with low friction between the solid and the die wall. Further, lubricants can generally increase the powder flowability. The lubricant is preferably a stearate or fatty acid, more preferably an earth alkali metal stearate, such as magnesium stearate. The lubricant is suitably present in an amount of 0 to 2 wt%, preferably of about 0.1 to 1.0 wt%, based on the total weight of the dosage form.

Glidants (h) can also be used to improve the flowability. Traditionally, talc was used as glidant, but is nowadays nearly fully replaced by colloidal silica (for example Aerosil^{®}). Preferably, the glidant can be present in an amount of 0 to 3 wt%, more preferably 0.1 to 2.5 wt%, in particular 0.25 to 2.0 wt%, based on the total weight of the dosage form.

It lies in the nature of pharmaceutical excipients that they sometimes can perform more than one function in a pharmaceutical formulation. In this regard it is generally noted that due to the nature of pharmaceutical excipients it cannot be excluded that a certain compound meets the requirements of more than one of components (c) to (h). However, in order to enable an unambiguous distinction, it is preferred in the present application that one and the same pharmaceutical compound can only function as one of the compounds (c) to (h). For example, if microcrystalline cellulose functions as a filler (d), it cannot additionally function as a disintegrant (f), even though microcrystalline cellulose also exhibits a certain disintegrating effect.

The oral dosage form of the present invention can preferably comprise
40 to 65 wt% vemurafenib hydrochloride, preferably 43 to 62 wt% vemurafenib hydrochloride,
30 to 45 wt% HPMC-AS, preferably 32 to 42 wt% HPMC-AS,
0 to 2 wt% surfactant, preferably 0.1 to 1.5 wt% surfactant,
0 to 25 wt% filler, preferably 1 to 20 wt% filler,
0 to 10 wt% binder, preferably 0.2 to 8 wt% binder,
0 to 15 wt% disintegrant, preferably 1 to 12 wt% disintegrant,
0 to 2 wt% lubricant, preferably 0.1 to 1.0 wt% lubricant and
0 to 3 wt% glidant, preferably 0.1 to 2.5wt% glidant,
based on the total weight of the oral dosage form.

In a preferred embodiment of the invention the dosage form of the present invention can preferably comprise vemurafenib hydrochloride in an amount from 240 to 550 mg.

In a more preferred embodiment the dosage form of the present invention can preferably comprise vemurafenib hydrochloride in an amount from 240 to 270 mg, even more preferably 255 to 265 mg, in particular 258 mg. The amounts refer to the vemurafenib salt but not to the amounts of free base.

In an alternative more preferred embodiment the dosage form of the present invention can preferably comprise vemurafenib hydrochloride in an amount of 500 to 550 mg, even more preferably 510 to 530 mg, in particular about 516 mg.. The amounts refer to the vemurafenib salt but not to the amounts of free base.

In a preferred embodiment the total weight of the dosage form is from 400 to 700 mg, preferably from 450 to 650 mg, in particular when 240 to 270 mg vemurafenib hydrochloride are contained in the dosage form. In an alternative preferred embodiment the total weight of the dosage form is from 800 to 1400 mg, preferably from 900 to 1300 mg, in particular when 500 to 550 mg vemurafenib hydrochloride are contained in the dosage form.

In a still further embodiment of the present invention the oral dosage form can be a solid oral dosage form, preferably a capsule or a tablet, more preferably a tablet, in particular for peroral use. Alternatively, the solid oral dosage form can be filled as powder or granulate into devices like sachets or stick-packs.

The present invention further relates to a method for producing a premix according to the invention. Hence, a further subject of the present invention is a method for preparing a premix comprising vemurafenib hydrochloride and HPMC-AS, wherein the weight ratio of vemurafenib as free base to HPMC-AS is from 1:1 to 2:1 comprising the steps of
(i) providing vemurafenib hydrochloride and HPMC-AS,
(ii) milling the mixture from step (i) and
(iii) optionally sieving the mixture from step (ii).

Generally, the comments made above for vemurafenib hydrochloride and HPMC-AS can also apply to the method of the present invention.

In step (i) vemurafenib hydrochloride and HPMC-AS are provided. Vemurafenib hydrochloride can preferably be provided in crystalline form. Further, vemurafenib hydrochloride can be provided in micronized form. The step of micronization can preferably be carried out by milling, such as in an air jet mill. Preferably, the average particle size D₅₀ of vemurafenib is from 1 to 25 µm, preferably from 2 to 15 µm and can be determined as described above. Optionally, vemurafenib hydrochloride and HPMC-AS can be blended in order to provide a composition having a homogenous distribution of vemurafenib hydrochloride within the resulting blend comprising vemurafenib hydrochloride and HPMC-AS. Blending can be carried out with conventional mixing devices, e.g. in a free-fall mixer like Turbula^{®} T10B (Bachofen AG, Switzerland). Blending can be carried out e.g. for 1 minute to 30 minutes, preferably for 2 minutes to less than 10 minutes.

In step (ii) milling of the mixture from step (i) can preferably be performed in conventional milling apparatuses, such as in a ball mill, air jet mill, pin mill, classifier mill, cross beater mill, disk mill, mortar grinder or a rotor mill. A ball mill is preferably used.

The milling time is preferably 5 minutes to 6 hours, preferably 10 minutes to 4 hours, more preferably 15 minutes to 2 hours.

In a preferred embodiment the milling conditions in step (ii) can preferably be selected such that the vemurafenib hydrochloride is in a non-crystalline form, i.e. that the XRD of the milled mixtures does not reveal any clearly defined interference patterns of vemurafenib hydrochloride, but at most only a few diffuse interferences with small diffraction angles.

It is preferred that the optional step (iii) of sieving the mixture of step (ii) can be carried out with a sieve having a mesh size of 25 to 1000 µm, preferably 50 to 800 µm, especially 100 to 600 µm.

Further, the subject of the present invention relates to a method for preparing the oral dosage form of the invention comprising the steps:
(i) providing vemurafenib hydrochloride and HPMC-AS,
(ii) milling the mixture from step (i),
(iii) optionally sieving the mixture from step (ii),
(iv) optionally adding further excipient(s) to the mixture of step (ii) or step (iii),
(v) optionally granulating the mixture of step (ii), step (iii) or step (iv) and
(vi) processing the mixture of step (ii), step (iii), or step (iv) or the granulates of step (v) into an oral dosage form.

In steps (i) to (iii) a premix according to the present invention is provided, i.e. all the above process steps (i), (ii) and (iii) leading to the present premix also apply to the process for preparing the present oral dosage form.

In step (iv) additional further excipient(s) and/or further pharmaceutically active agent can optionally be added to the mixture of step (ii) or step (iii). During or after the addition of the optional excipients and/or further pharmaceutically active agent the resulting mixture can preferably be blended. Blending can be carried out with the conventional mixing devices as described above. The excipients can preferably be selected from the excipients (c) to (h) as described above.

In step (v) the mixture of step (ii), step (iii) or step (iv) can be optionally granulated.

"Granulating" is generally understood to mean the formation of relatively coarse or granular aggregate material as a powder by assembling and/or aggregating finer powder particles (agglomerate formation or build-up granulation) and/or the formation of finer granules by breaking up coarser aggregates (disintegration or break-down granulation).

Granulation can conventionally mean wet or dry granulation.

Dry granulation, which is preferred, is generally carried out by using pressure or temperature. In a preferred embodiment of the invention, granulating the mixture from step (ii), step (iii) or step (iv) can be performed for example by "slugging" using a large heavy-duty rotary press and breaking up the slugs into granulates with a hammer mill or by roller compaction using for example roller compactors by Powtec or Alexanderwerk. The granulates are then optionally screened.

In step (vi) the mixture of step (ii), step (iii) or step (iv) or the granulates of step (v) can be processed into a solid oral dosage form. Processing the mixture of step (ii), step (iii) or step (iv) or the granulates from step (v) into a solid oral dosage form can preferably comprise filling said mixture of step (ii), step (iii) or step (iv) or said granulates from step (v) into capsules, preferably hard gelatine capsules.

More preferred, processing the mixture of step (ii), step (iii) or step (iv) or the granulates from step (v) into tablets can be carried out by compressing said formulation on a rotary press, e.g. on a Fette^{®} (Fette GmbH, Germany) or a Riva^{®} piccola (Riva, Argentina). The main compression force can range from 1 to 50 kN, preferably from 3 to 40 kN. The resulting tablets can have a hardness of 30 to 400 N, more preferred of 50 to 250 N, particularly preferably of 30 to 180 N, more preferably 40 to 150 N, wherein the hardness can be measured according to Ph.Eur. 6.0, Chapter 2.9.8. For the optional filling of the formulation into capsules dependent dosing systems (for example an auger) or preferably independent dosing systems (for example MG2, Matic (IMA)) can be used.

Further, the dosage form, preferably the tablet, of the invention preferably has a content uniformity, i.e. a content of active agent(s) which lies within the concentration of 90 to 110%, preferably 95 to 105%, especially preferred from 98 to 102% of the average content of the active agents(s). The "content uniformity" is determined with a test in accordance with Ph. Eur., 6.0, Chapter 2.9.6. According to that test, the content of the active agent of each individual tablet out of 20 tablets must lie between 90 and 110%, preferably between 95 and 105%, especially between 98 and 102% of the average content of the active agent(s). Therefore, the content of the active drugs in each tablet of the invention differs from the average content of the active agent by at most 10%, preferably at most 5% and especially at most 2%.

In addition, the resulting tablet preferably has a friability of less than 5%, particularly preferably less than 2%, especially less than 1%. The friability is determined in accordance with Ph. Eur., 6.0, Chapter 2.9.7. The friability of tablets generally refers to tablets without coating.

The oral dosage form the invention may be a peroral tablet which can be swallowed unchewed. The tablet can preferably be film coated.

Generally, film coatings that do not affect the release of the active agent(s) and film coatings affecting the release of the active agent(s) can be employed with tablets according to invention. The film coatings that do not affect the release of the active agent(s) are preferred.

Preferred examples of film coatings which do not affect the release of the active ingredient can be those including poly(meth)acrylate, methylcellulose (MC), hydroxypropyl methylcellulose (HPMC), hydroxypropyl cellulose (HPC), hydroxyethyl cellulose (HEC), polyvinylpyrrolidone (PVP) and mixtures thereof. These polymers can have a weight-average molecular weight of 10,000 to 150,000 g/mol.

In an alternative preferred embodiment, the film coating can affect the release of the active agent. Examples for film coatings affecting the release of the active agent are gastric juice-resistant film coatings and retard coatings.
In a preferred embodiment the film can have a thickness of 2 µm to 150 µm, preferably from 10 to 100 µm, more preferably from 20 to 60 µm.

In a preferred embodiment the dosage form of the invention is for modified release. In that case the release profile of the pharmaceutical formulation, preferably of the tablet, according to USP method (apparatus 2; paddle, 900 ml of 1% hexadecyltrimethylammonium bromide (HTAB) in 50 mmol/L phosphate buffer at pH 6.8 and 37°C, 100 rpm) after 2 hours indicates a content release of 0 to 90%, preferably of 10 to 80%, further preferably 15 to 75%, more preferably 20 to 50% and particularly of 25 to 40%.

In a more preferred embodiment of the invention the dosage form is for immediate release. In that case the release profile of the pharmaceutical formulation, preferably of the tablet, according to USP method (apparatus 2; paddle, 900 ml of 1% hexadecyltrimethylammonium bromide (HTAB) in 50 mmol/L phosphate buffer at pH 6.8 and 37°C, 100 rpm) after 15 minutes indicates a content release of at least 70%, preferably at least 80%, especially from 85 to 99 %.

Further, the invention relates to a premix according to the invention or the dosage form according to the invention for use in the treatment of cancer, preferably colorectal cancer, ovarian carcinoma, thyroid cancer and/or malignant melanoma. For the pharmaceutical composition used in the before-mentioned treatment the same applies as to the premix and the oral dosage form as described above in the text.

### Experimental Part

### Analytical Methods:

The samples were examined by X-ray powder diffraction.

### X-Ray Powder Diffraction

The measurements were performed as follows: The samples were measured on a D8 Advance powder X-ray diffractometer (Bruker-AXS, Karlsruhe, Germany) in a PMMA sample holder rotating at 20 rpm during the measurement (Bragg-Brentano geometry). Further conditions for the measurements are summarized below. The raw data were analysed with the program EVA (Bruker-AXS, Karlsruhe, Germany).

| | |
|---|---|
| Detector: | Vantec-1 3°2θ |
| Radiation: | Cu K_{α} (1.5418Å) |
| Monochromator: | None |
| Second ß filter: | Ni 0.1 mm |
| Start angle: | 2° |
| End angle: | 55° |
| Measurement time: | 11 min |
| Step: | 0.016° 2θ |

### Solubility:

The medium for determining the solubility is an aqueous 50mM KH₂PO₄-solution supplemented with 2.0% (v/v) d-alpha tocopheryl polyethylene glycol 1000 succinate (TPGS) at pH 6.5.

The following procedure was used:
At 23°C the sample was given into a glass vial and 1 ml of the above medium was added. The vial was stirred 15 sec on a Reax, the sample was filtered through a PTFE-filter and spontaneous dissolution was analyzed by HPLC/UV.

### EXAMPLES

### Example 1:

500 mg of crystalline vemurafenib hydrochloride and 465.3 mg of HPMC-AS were milled in a "Fritsch pulverisette" for 1 hour at 400 rpm with 10 balls each having a diameter of 10 mm.

### Example 2:

500 mg of crystalline vemurafenib hydrochloride and 232.73 mg of HPMC-AS were milled in a "Fritsch pulverisette" for 1 hour at 400 rpm with 10 balls each having a diameter of 10 mm.

### Reference Example 1:

A solid dispersion comprising vemurafenib free base and HPMC-AS in a weight ratio of 3:7 was prepared according to Examples 1 to 5 of WO 2011/057974.

### Reference Example 2:

A solid dispersion comprising vemurafenib free base and HPMC-AS in a weight ratio of 1:1 was prepared according to Examples 1 to 5 of WO 2011/057974.

### Reference Example 3:

500 mg of crystalline vemurafenib free base and 500 mg of HPMC-AS were milled in a "Fritsch pulverisette" for 1 hour at 400 rpm with 10 balls each having a diameter of 10 mm.

### RESULTS

The solubility of the samples was determined according to the precedure as mentioned above. The results are specified in Table A.

**Table A**

| **Sample** | **sample weight [mg]** | **solvent [µl]** | **solubility [mg/ml]** |
|---|---|---|---|
| Example 1 | 14.54 | 1000 | 2.864 |
| Example 2 | 11.05 | 1000 | 2.914 |
| Reference Example 1 | 23.24 | 1000 | 1.611 |
| Reference Example 2 | 14.00 | 1000 | 0.137 |
| Reference Example 3 | 14.33 | 1000 | 0.210 |

As can be seen, the solubility of the present Examples is significantly higher especially compared to Reference Examples 2 and 3. Further, compared to Reference Example 1 a proportionally small amount of HPMC-AS is used with regard to the active agent and nevertheless a higher solubility of the present premix was achieved. In view of the before mentioned the present premix allows formulating a dosage form having a higher drug load or alternatively a dosage form with the same content of active agent but being significantly smaller in size.

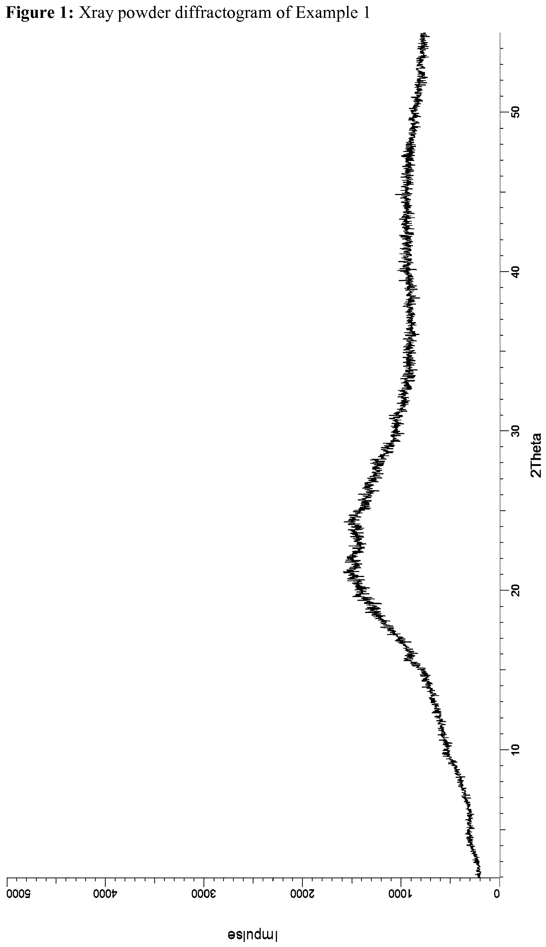

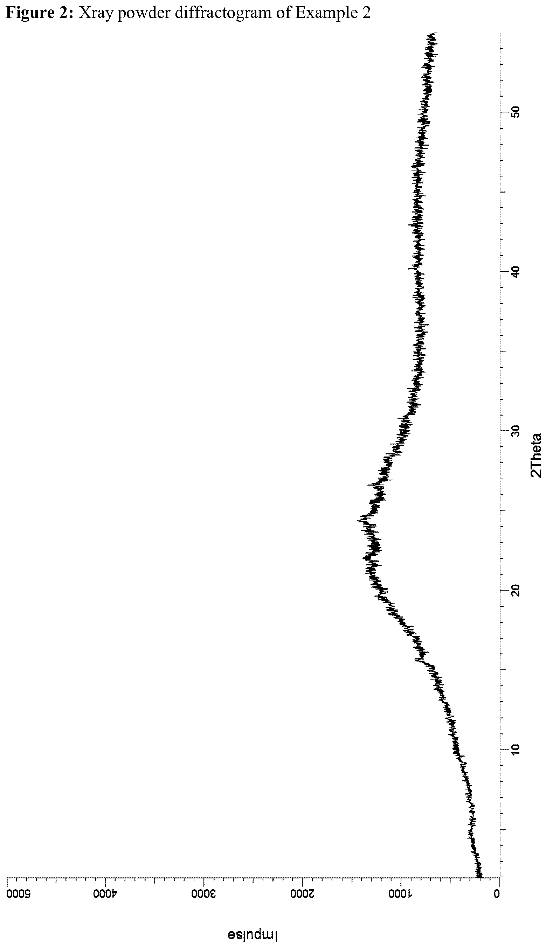

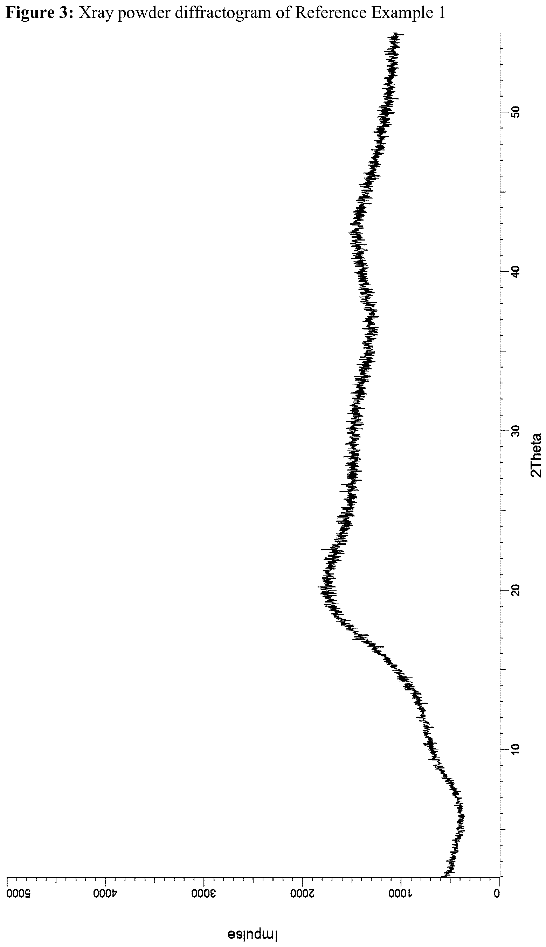

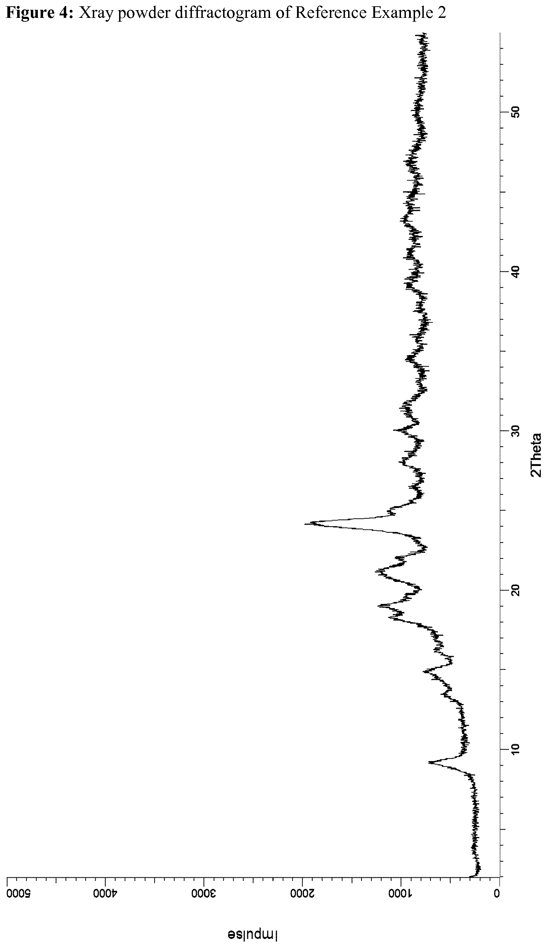

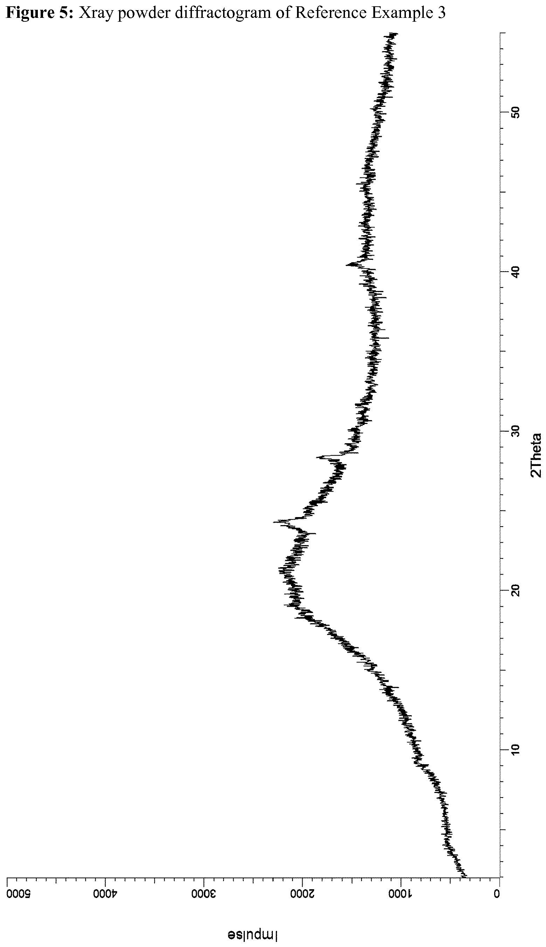

Further, X-ray powder diffraction was conducted for Example 1 and Example 2. As can be seen from Figures 1 and 2, the corresponding X-ray powder diffractograms do not show any defined interferences. Thus, in the present premix vemurafenib is present in amorphous form.

In Reference Example 3 no amorphous vemurafenib could be obtained. Instead, X-ray powder diffraction showed defined interferences of crystalline vemurafenib. In other words, it is not possible to increase the drug load by mereley reducing the amount of HPMC-AS.

In the present invention it was unexpectedly found that the change from the free base to the hydrochloric salts increases the non-crystalline properties. Such finding is particularly unexpected since salts often have better crystallizing properties than the free salt.

## Claims

1. Premix comprising
a) vemurafenib hydrochloride, and
b) HMPC-AS,
wherein the weight ratio of vemurafenib as free base to HMPC-AS is from 1:1 to 2:1.

2. Premix according to claim 1, wherein the vemurafenib hydrochloride is present in a non-crystalline form.

3. Premix according to claim 1 or 2, wherein the premix has an average particle size D₅₀ of 1 to 50 µm.

4. Oral dosage form comprising the premix according to any one of claims 1 to 3.

5. Oral dosage form according to claim 4, wherein the dosage form comprises 40 to 65 wt% vemurafenib hydrochloride,
30 to 45 wt% HPMC-AS,
0 to 2 wt% surfactant
0 to 10 wt% binder,
0 to 15 wt% disintegrant,
0 to 2 wt% lubricant and
0 to 3 wt% glidant,
based on the total weight of the oral dosage form.

6. Oral dosage form according to any one of claims 4 to 5, wherein the dosage form comprises 250 mg to 270 mg or 500 mg to 540 mg vemurafenib hydrochloride.

7. Oral dosage form according to any one of claims 3 to 6, wherein the dosage form has a total weight from 400 mg to 700 mg or from 800 mg to 1400 mg.

8. Oral dosage form according to any one of claims 3 to 7, wherein the content release of the dosage form after 15 minutes is 70 % to 100 % determined by USP method (apparatus 2; paddle, 900 ml of 1% hexadecyltrimethylammonium bromide (HTAB) in 50 mmol/L phosphate buffer at pH 6.8 and 37°C, 100 rpm).

9. Method for preparing the premix according to claims 1 to 3, comprising the steps of
(i) providing vemurafenib hydrochloride and HPMC-AS,
(ii) milling the mixture from step (i) and
(iii) optionally sieving the mixture from step (ii)

10. Method according to claim 9, wherein the milling conditions in step (ii) are selected such that the vemurafenib hydrochloride is obtained after milling in a non-crystalline form.

11. Method for preparing an oral dosage form according to any one of claims 4 to 8 comprising the steps of
(i) providing vemurafenib hydrochloride and HPMC-AS,
(ii) milling the mixture from step (i),
(iii) optionally sieving the mixture from step (ii),
(iv) optionally adding further excipient(s) to the mixture of step (ii) or step (iii),
(v) optionally granulating the mixture of step (ii), step (iii) or step (iv) and
(vi) processing the mixture of step (ii), step (iii) or step (iv) or the granulates of step (v) into an oral dosage form.

12. Dosage form according to any one of claims 4 to 8 for use in the treatment of cancer, preferably colorectal cancer, ovarian carcinoma, thyroid cancer and/or malignant melanoma.

## Patentansprüche

1. Vormischung enthaltend,
a) Vemurafenib-Hydrochlorid, und
b) HMPC-AS,
wobei das Gewichtsverhältnis von Vemurafenib als freie Base zu HMPC-AS von 1:1 zu 2:1 beträgt.

2. Vormischung gemäß Anspruch 1, wobei das Vemurafenib-Hydrochlorid in einer nichtkristallinen Form vorliegt.

3. Vormischung gemäß Anspruch 1 oder 2, wobei die Vormischung eine durchschnittliche Teilchengröße D₅₀ von 1 bis 50 µm aufweist.

4. Orale Darreichungsform, welche die Vormischung gemäß einem der Ansprüche 1 bis 3 enthält.

5. Orale Darreichungsform gemäß Anspruch 4, wobei die Darreichungsform umfasst
40 bis 65 Gew.% Vemurafenib-Hydrochlorid,
30 bis 45 Gew.% HPMC-AS
0 bis 2 Gew.% oberflächenaktive Substanz,
0 bis 25 Gew.% Füllstoff,
0 bis 10 Gew.% Bindemittel,
0 bis 15 Gew.% Sprengmittel,
0 bis 2 Gew.% Schmiermittel und
0 bis 3 Gew.% Gleitmittel,
basierend auf dem Gesamtgewicht der oralen Darreichungsform.

6. Orale Darreichungsform gemäß einem der Ansprüche 4 bis 5, wobei die Darreichungsform 250 mg bis 270 mg oder 500 mg bis 540 mg Vemurafenib-Hydrochlorid umfasst.

7. Orale Darreichungsform gemäß einem der Ansprüche 3 bis 6, wobei die Darreichungsform ein Gesamtgewicht von 400 mg bis 750 mg oder von 800 mg bis 1400 mg aufweist.

8. Orale Darreichungsform gemäß einem der Ansprüche 3 bis 7, wobei die Inhaltsfreisetzung der Darreichungsform nach 15 Minuten 70% bis 100% beträgt, wenn gemäß der USP-Methode (Vorrichtung 2, Paddel, 900 ml von 1% Hexadecyltrimethylammoniumbromid (HTAB) in 50 mmol/L Phosphatpuffer bei pH 6,8 und 37°C, 100 Umdrehungen pro Minute) gemessen wird.

9. Verfahren zum Herstellen der Vormischung gemäß den Ansprüchen 1 bis 3, umfassend die Schritte
(i) zur Verfügung stellen von Vemurafenib-Hydrochlorid und HPMC-AS,
(ii) Mahlen der Mischung von Schritt (i) und
(iii) optionales Sieben der Mischung von Schritt (ii).

10. Verfahren gemäß Anspruch 9, wobei die Bedingungen zum Mahlen in Schritt (ii) so gewählt werden, dass das nach dem Mahlen erhaltende Vemurafenib-Hydrochlorid in einer nichtkristallinen Form vorliegt.

11. Verfahren zum Herstellen einer oralen Darreichungsform gemäß einem der Ansprüche 4 bis 8, umfassend die Schritte
(i) zur Verfügung stellen von Vemurafenib-Hydrochlorid und HPMC-AS,
(ii) Mahlen der Mischung von Schritt (i),
(iii) optionales Sieben der Mischung von Schritt (ii),
(iv) optionales Zugeben von weiteren Hilfsstoff(en) zu der Mischung von Schritt (ii) oder Schritt (iii),
(v) optionales Granulieren der Mischung von Schritt (ii), Schritt (iii) oder Schritt (iv) und
(vi) Verarbeiten der Mischung von Schritt (ii), Schritt (iii) oder Schritt (iv) oder den Granulaten von Schritt (v) zu einer oralen Darreichungsform.

12. Darreichungsform gemäß einem der Ansprüche 4 bis 8 zur Verwendung in der Behandlung von Krebs, vorzugsweise Darmkrebs, Ovarialkarzinom, Schilddrüsenkrebs und/oder malignem Melanom.

## Revendications

1. Prémélange comprenant
a) du chlorhydrate de vemurafenib et
b) du HPMC-AS,
dans lequel le rapport pondéral entre le vemurafenib en tant que base libre et le HMPC-AS est de 1:1 à 2:1.

2. Prémélange selon la revendication 1, dans lequel le chlorhydrate de vemurafenib est présent sous une forme non cristalline.

3. Prémélange selon la revendication 1 ou 2, dans lequel le prémélange a une taille de particule moyenne D₅₀ de 1 à 50 µm.

4. Forme posologique orale comprenant le prémélange selon l'une quelconque des revendications 1 à 3.

5. Forme posologique orale selon la revendication 4, dans laquelle la forme posologique comprend
40 à 65% en poids de chlorhydrate de vemurafenib,
30 à 45% en poids de HPMC-AS,
0 à 2% en poids de tensioactif,
0 à 10% en poids de liant,
0 à 15% en poids d'agent de délitement,
0 à 2% en poids de lubrifiant et
0 à 3% en poids d'agent de glissement,
par rapport au poids total de la forme posologique orale.

6. Forme posologique orale selon l'une quelconque des revendications 4 à 5, dans laquelle la forme posologique comprend de 250 mg à 270 mg ou de 500 mg à 540 mg de chlorhydrate de vemurafenib.

7. Forme posologique orale selon l'une quelconque des revendications 3 à 6, dans laquelle la forme posologique a un poids total de 400 mg à 700 mg ou de 800 mg à 1400 mg.

8. Forme posologique orale selon l'une quelconque des revendications 3 à 7, dans laquelle la libération du contenu de la forme de dosage après 15 minutes est de 70% à 100% tel, déterminé conformément à la méthode selon USP (appareil 2; pagaie, 900 ml de bromure d'hexadécyltriméthylammonium (CCFH) de 1% dans 50 mmol / L de tampon phosphate à pH 6,8 et à 37 °C, 100 tours/minute (rpm)).

9. Procédé pour la préparation du prémélange selon les revendications 1 à 3, comprenant les étapes consistant à
(i) fournir du chlorhydrate de vemurafenib et du HPMC-AS,
(ii) broyer le mélange de l'étape (i) et
(iii) facultativement tamiser le mélange de l'étape (ii).

10. Procédé selon la revendication 9, dans lequel les conditions de broyage à l'étape (ii) sont choisis de telle sorte que le chlorhydrate de vemurafenib est obtenu après broyage sous une forme non cristalline.

11. Procédé de préparation d'une forme posologique orale selon l'une quelconque des revendications 4 à 8, comprenant les étapes consistant à
(i) fournir du chlorhydrate de vemurafenib et du HPMC-AS,
(ii) broyer le mélange de l'étape (i) et
(iii) facultativement tamiser le mélange de l'étape (ii),
(iv) facultativement ajouter un ou plusieurs excipient(s) supplémentaire(s) au mélange de l'étape (ii) ou l'étape (iii),
(v) facultativement granuler le mélange de l'étape (ii), de l'étape (iii) ou de l'étape (iv), et
(vi) transformer le mélange de l'étape (ii), de l'étape (iii) ou de l'étape (iv) ou les granulés de l'étape (v) en une forme posologique orale.

12. Forme posologique orale selon l'une quelconque des revendications 4 à 8 pour utilisation dans le traitement d'un cancer, de préférence d'un cancer colorectal, d'un cancer de l'ovaire, d'un cancer de la thyroïde et / ou de mélanome malin.
